# EUROPÄISCHE PATENTSCHRIFT

(11) **EP 0 891 741 B1**
(45) Veröffentlichungstag und Bekanntmachung des Hinweises auf die Patenterteilung: **02.05.2002**
(21) Anmeldenummer: 98113074.3
(22) Anmeldetag: 14.07.1998
(51) Int. Cl.: A61B 5/0408, A61N 1/04

(54) **Biomedizinische Elektrode und Verfahren zu deren Herstellung**
Biomedical electrode and method for its manufacture
Electrode biomédicale et son procédé de fabrication

(30) Priorität: 18.07.1997 DE 19730811
(43) Veröffentlichungstag der Anmeldung: 20.01.1999
(73) Patentinhaber: Tyco Healthcare Deutschland Manufacturing GmbH, 93333 Neustadt/Donau (DE)
(72) Erfinder: Anz, Johannes, 38820 Halberstadt (DE); Barth, Hans-Günther, 38820 Halberstadt (DE); Malkowsky, Andrea, 38828 Wegeleben (DE); Barth, Hannelore, 38820 Halberstadt (DE)
(74) Vertreter: Tragsdorf, Bodo, Dipl.-Ing.

(56) Entgegenhaltungen:
- EP-A- 0 619 094
- DE-A- 4 336 302
- US-A- 4 522 211

## Beschreibung

Die Erfindung betrifft eine biomedizinische Elektrode, die zur Übertragung elektrischer Signale von der Haut zu einem elektromedizinischen Geräte (Passivelektroden) oder zur Übertragung externer elektrischer Signale zur Haut (Aktivelektroden) geeignet ist und bei der die von der Hautfläche abweisende Seite der Elektrode aus einer metallisch leitfähig beschichteten Polymerbahn besteht. Ferner bezieht sich die Erfindung auf ein geeignetes Herstellungsverfahren für die Elektroden.

Auf die Haut aufklebbare Elektroden, bestehend aus einem flexiblen Trägermaterial, z.B. einer geschlossenzelligen Polymerschaumbahn, das eine Öffnung zur Aufnahme eines elektrisch leitenden Gels zur Kontaktierung mit der Haut des Patienten aufweist, und aus einer Polymerbahn an der von der Hautoberfläche abweisenden Seite, die an ihrer Unterseite mit einer leitfähigen Beschichtung versehen ist, sind bereits bekannt. Eine derartige Elektrode ist z.B. in der WO 96/32058 beschrieben. Die leitfähige Polymerbahn besteht aus einem nichtklebenden Etikett, das aus elektrisch leitfähigem Material hergestellt ist oder auf seiner Unterseite eine elektrisch leitende Schicht in Form einer Leiterbahn aufweist. Auf die Unterseite der Etiketten wird eine Klebschicht aufgetragen, die nicht daueraktiv ist. Gegebenenfalls wird die Klebschicht erst nach dem Bedrucken aufgetragen.

Gemäß den verschiedenen dargestellten Ausführungsvarianten überdecken die Etiketten nur einen relativ kleinen Bereich des Trägermaterials. Dadurch ergeben sich Probleme beim Befestigen der Etiketten auf dem Trägermaterial. Der Bereich der Etiketten, der die Öffnung für den Gelkörper abdeckt, darf nicht mit aktivem Kleber in Berührung kommen, da dadurch die Leitfähigkeit beeinträchtigt wird. Der Einsatz spezieller Kleber erfordert einen zusätzlichen Aufwand und wirkt sich nachteilig auf die Herstellungstechnologie für die Elektroden aus. Das leitfähige Etikett bzw. die auf das Etikett aufgedruckten Leiterbahnen gewährleisten keine optische Transparenz im Bereich des Gelkörpers. Der Einsatz eines durch Wärme aktivierbaren Klebstoffes, z.B. eines Schmelzklebstoffes, hat den Nachteil, daß bei der Montage der Klebstoff mittels Wärme auch dort aktiviert werden muß, wo der Leitlack die Verbindung zwischen Sensor und Abgriff herstellt. Da das Etikett aus thermoplastischem Material besteht und für einen sicheren Verbund zum Trägermaterial auch eine gewisse Druckkraft aufgebracht werden muß, wird in diesen derart behandelten Zonen der in geringer Schichtdicke ohne eigene Festigkeit aufgebrachte Leitlack, der ebenfalls mit einem thermoplastischen Lackrohstoff gebunden sein kann, durch Fließvorgänge in seinem Querschnitt gestört oder gar beschädigt.

Weitere Knickbelastungen wirken sich dann nachteilig auf die Funktionsfähigkeit der Elektrode aus. Ein weiterer wesentlicher Nachteil dieser Elektrode besteht darin, daß der elektrische Abgriff der Elektrode ausschließlich über das Etikett erfolgt. Der Abgriffbereich ist erhöhten mechanischen Belastungen ausgesetzt. Dadurch besteht die Gefahr, daß durch auftretende Knickvorgänge die Leitfähigkeit der Leiterbahn unterbrochen werden kann und Funktionsstörungen auftreten.

Außerdem wird eine gewisse Eigensteifigkeit des Ableitungskabels nicht ausreichend abgefangen, so daß sich Kabel und Druckknopfanschluß (Adapter) leicht verdrehen. Im Falle steiferer Etiketten wird die Elektrode selbst steif, und damit anfällig gegen Bewegungsartefakte und vorzeitiges Ablösen von der Haut.

Auch bei einem scharf gefalteten Etikett treten die vorgenannten Nachteile erst recht auf. Die Anordnung des Abgriffbereiches der Elektrode an dem Etikett wirkt sich nachteilig auf den Herstellungsprozeß der Elektroden aus.

Aus der DE 43 11 354 C1 ist eine Körperelektrode bekannt, die aus einem kreisförmigen Elektrodenteil und einem Leitungsteil besteht, das ca. 20 bis 60 cm lang und nur einige Millimeter breit ist. Auf das Elektrodenteil ist ein Sensor aus einem Leitlack aufgedruckt, der aus einem Kreisring und acht radial ausgerichteten, speichenförmigen Stegen besteht, die sich im Zentrum des Kreisringes treffen. Mit dem Sensor ist eine gedruckte Anschlußleitung verbunden, die sich über die gesamte Länge des Leitungsteiles erstreckt und an ihrem Ende zur Ausbildung einer Kontaktfläche verbreitert ist. Durch das lange Leitungsteil wird vermieden, daß auftretende Biegeund Drehmomente auf das Elektrodenteil übertragen werden. Diese Elektrode wird bevorzugt als Neonatenelektrode eingesetzt oder für Patienten, die bettlägerig sind, wobei die Elektrode direkt an den Monitor angeschlossen ist. Diese Elektrode ist in ihrer Herstellung kostenaufwendig und für Anwendungsfälle, wie z.B. bei Langzeit-EKG, wo eine normale Bewegungsfreiheit des Patienten gewährleistet sein soll, ungeeignet.

Da auf den Elektrodenteil infolge des langen Leitungsteiles keine Biege- und Drehmomente übertragen werden, dient die unterschiedliche Druckdichte des Leitlackes im Elektrodenteil ausschließlich zur Bildung optischer Fenster, um die applizierte Hautpartie während der Messung beobachten zu können.

Ein weiterer Nachteil dieses Leitlackmusters ist ein relativ großer Verbrauch an Leitlack. Die auf Edelmetallen basierenden Leitlacke sind sehr kostenintensiv, so daß sich ein hoher Leitlackverbrauch deutlich auf die Kosten bzw. den Preis der Elektroden auswirkt.

Ein kostengünstiges Verfahren zur Herstellung biomedizinischer Elektroden ist aus der DE 43 36 300 C2 bekannt.

Zur Herstellung der Elektroden sind folgende Verfahrensschritten vorgesehen:
a) eine einseitig haftklebstoffbeschichtete Bahn mit einer silikonisierten Papierabdeckung, die mindestens in einem Abstand von 5 bis 25 mm vom Rand längs durchgetrennt ist, wird, mit der haftklebstoffbeschichteten Seite nach oben zeigend, taktweise zu einer Schneidvorrichtung transportiert,
b) entsprechend dem Taktschritt der Transportvorrichtung werden mittels der Schneidvorrichtung Aussparungen in die Bahn eingebracht, die mindestens der Größe der Deckfläche des später zu bildenden Gelkörpers entsprechen,
c) die Aussparung wird von unten mit einer klebfähigen Folie abgedeckt, wobei der die Aussparung überragende Teil der Folie fest mit der Bahn verklebt wird,
d) anschließend wird in die aufgesetzte Folie mittig eine Öffnung eingebracht, in die die Eyeletnase eingeführt und von unten das Stud aufgesetzt wird, derart, daß die Folie zwischen dem Eyelet und dem Stud fest eingeklemmt ist,
e) es wird eine Kavität gebildet, in die mittels einer Dosiereinrichtung eine vorbereitete polymerisierbare Reaktionsmischung eingefüllt wird,
f) danach durchläuft die Bahn mit der Reaktionsmischung eine UV-Belichtungsstrecke, wobei die Reaktionsmischung zu einem Gelkörper unter Normalbedingungen polymerisiert,
g) nach dem Verlassen der UV-Belichtungsstrecke wird die die Bahn abdeckende silikonisierte Papierbahn entfernt und die klebrige Oberseite von Bahn sowie Gelkörper mit einer antiadhäsiven Abdeckung versehen und
h) die fertige Elektrode ausgestanzt.

Bei der so hergestellten Elektrode befindet sich der Abgriffbereich direkt über dem Gelkörper, der Nietsensor des Elektrodenanschlußknopfes steht mit dem Gelkörper in Berührungskontakt.

Der Erfindung lag die Aufgabe zugrunde, eine biomedizinische Elektrode mit einer dezentralen Ankopplung an übliche Leitungssysteme und mit einem Leitlackmuster zur Übertragung der elektrischen Signale vom Leitgelkörper an einen Anschluß außerhalb des Leitgelkörpers zu schaffen, die während der Applikation hohen mechanischen Belastungen ausgesetzt werden kann und deren Leitlackmuster so ausgebildet ist, daß eine sichere und artefaktfreie Signalübertragung gewährleistet ist sowie eine ausreichende optische Transparenz zur Beobachtung der applizierten Hautpartie, und die kostengünstig herstellbar ist. Ferner war es Aufgabe der Erfindung ein geeignetes, kostengünstiges Herstellungsverfahren zu schaffen.

Erfindungsgemäß wird die Aufgabe durch die im Anspruch 1 angegebenen Merkmale gelöst. Geeignete Ausgestaltungsvarianten der Elektrode sind in den Ansprüchen 2 bis 7 sowie 11 angegeben. Die erfindungsgemäße Herstellung der Elektroden erfolgt nach drei unterschiedlichen Verfahren, deren Merkmale in den Ansprüchen 8, 9 und 10 beschrieben sind.

Die vorgeschlagene Elektrode besitzt eine seitlich abstehende Abgrifflasche, in der der Anschluß für die Abgriffelemente für den externen Signalanschluß angeordnet ist. Die Abgrifflasche besteht aus dem Trägermaterial der Polymerschaumbahn und der leitlackbeschichteten Polymerbahn. An der zu Haut zugewandten Seite ist die Abgrifflasche mit einer nichtklebenden Abdeckung versehen. Die auf der Oberseite der Polymerschaumbahn aufgeklebte Polymerbahn ist ganzflächig mit dem Trägermaterial, der Polymerschaumbahn, verbunden. Dadurch wird gewährleistet, daß der Abgriff der Elektrode problemlos den beim Gebrauch auftretenden erhöhten mechanischen Belastungen ausgesetzt werden kann. Die Abgrifflasche besitzt eine ausreichende Steifigkeit, so daß Verdrehungen des Ableitungskabels abgefangen werden können und die Elektrode trotzdem sicher auf der Haut sitzt. Da der Abschnitt der Elektrode, der unmittelbar auf der Haut aufsitzt, nicht diese Steifigkeit wie die Abgrifflasche besitzt, ist diese Elektrode nicht anfällig gegen Bewegungsartefakte und vorzeitiges Ablösen von der Haut. Der Leitgelkörper der Elektrode ist transparent ausgebildet. Der Leitlack zwischen dem Leitgelkörper und der Abgriffstelle besteht aus einem Leitlackmuster unterschiedlicher Druckdichte. Die Druckdichte des Bereiches der Polymerbahn, die den Leitgelkörper abdeckt, ist so bemessen, daß mindestens zwei Drittel dieses Bereiches transparent sind. Die Ausbildung des Leitlackes als Muster und die dadurch mögliche optische Transparenz im Bereich des Leitgelkörpers ermöglichen die Beobachtung von Veränderungen der Haut während der Applikation der Elektrode. Der Bereich der Elektrode, der den Übergang vom Leitgelkörper zur Abgrifflasche bildet, ist mit einer wesentlich höheren Druckdichte ausgestattet. Die Druckdichte des Leitlackmusters in diesem Bereich ist mindestens um ein Drittel höher als die Druckdichte im Bereich des Leitgelkörpers. Dadurch wird gewährleistet, daß infolge der Biegebeanspruchungen der Abgrifflasche keine Kontaktunterbrechungen auftreten. Im Bereich um die Abgriffstelle weist das Leitlackmuster eine Dichte auf, die mindestens so hoch ist wie die im Bereich des Leitgelkörpers.

Durch die Anordnung einer Haftungslücke zwischen der Polymerbahn und der Polymerschaumbahn im Knickbereich und/oder der Perforierung der Polymerschaumbahn in diesem Bereich kann die Beweglichkeit der Abgrifflasche noch verbessert werden. Außerhalb des Bereiches, in dem sich der Leitgelkörper befindet, kann die Polymerbahn noch zusätzlich bedruckt werden, z.B. mit einem Logo, einem Bildmotiv, einer Typenbezeichnung und dgl.. Es kann ein gesonderter Druck aufgebracht werden oder das Leitlackmuster wird in diesem Bereich vom Druck her so ausgebildet. Das Leitlackmuster kann als Endlosdruck oder Einzeldruck ausgeführt sein. Eine laufende Kontrolle der Druckparameter kann z.B. durch einen gleichzeitigen Kontrolldruck ermöglicht werden.

Der Einzeldruck des Leitlackmusters ist kostengünstiger. Versuche haben gezeigt, daß es zweckmäßig ist, wenn auf die hautabgewandte Seite im Bereich der Abgrifflasche zusätzlich noch eine selbstklebende, flexible Bahn aufgebracht wird, die auch bedruckbar sein kann. Dadurch wird bei besonders stark beanspruchten Elektroden im Knickbereich die Gefahr einer Bruchneigung der Leitlacklinien ausgeschlossen. Durch diese zusätzliche Bahn, z.B. einer PP-Folie, wird auch ein Abdrehen oder Ausreißen des Abgriffelemtentes, z.B. einer Druckknopfverbindung, bei Verdrillung des Ableitkabels verhindert und eine Beschädigung der bedruckten Polymerbahn bei Verwendung von Krokodilklammem vermieden.

Die erfindungsgemäßen Elektroden weisen eine Vielzahl anwendungs- oder herstellungstechnischer Vorteile im Vergleich zu den bisher bekannten Elektroden auf.

Diese bestehen in folgendem:
Als vorteilhafte anwendungstechnische Parameter gelten a) gute Trageeigenschaften, insbesondere bei Langzeitanwendungen, bei denen die Elektrode über mehrere Tage auf der Haut verbleibt, b) eine hohe Signaltreue, auch bei körperlicher Bewegung bzw. externen Bewegungen der Kabelanschlüsse, c) die Möglichkeit, den Gelbereich insoweit optisch transparent zu gestalten, so daß z.B. eine visuelle Kontrolle der darunterbefindlichen Hautpartie oder optische Effekte durch Einfärben des Gelkörpers gestattet werden, d) ein Aufbau, der je nach Zweck eine praktisch beliebige Anpaßbarkeit der metallisch leitfähigen Sensorfläche in geometrischer und elektrotechnischer Hinsicht an die Belange der Signalableitung bzw. der Stromdichte und ihrer Verteilung gestattet, e) entsprechend eine beliebige Anpaßbarkeit des Gelableitkörpers an die Erfordernisse der Elektrode hinsichtlich ihrer geometrischen Gestalt und dem Typ des einzusetzenden Gels, f) die Möglichkeit, die Elektrode röntgentransluzent zu gestalten, g) gute praktische Handhabbarkeit, h) freie Gestaltbarkeit in Kontur und Layout, i) relativ lange Lagerstabilität, auch außerhalb der Verpackung und k) möglichst niedrige Umweltbelastung bei der Entsorgung. Dazu gehört ein Herstellungsverfahren, das, dem Niveau der marktgängigen vergleichbaren bisherigen Elektroden entsprechend, eine stabile, möglichst einfache und kostengünstige Fertigung ermöglicht. Schließlich rückt bei Einwegartikeln, die in hohen Stückzahlen verbraucht werden, die Entsorgungsproblematik immer mehr in den Vordergrund.

Elektroden für die Langzeit-EKG-Überwachung, die Überwachung von Intensivpatienten mit besonders schwierigen Anforderungen an das Elektrodenmaterial, die Ergometrie und Sportmedizin oder Aktivelektroden mit der Notwendigkeit einer gleichmäßigen Stromdichteverteilung sind typische Anwendungen. Darüber hinaus ergeben sich weitere Anwendungen aus dem Bereich der Aktiv- wie der Passivhautelektroden (beispielsweise Defibrillations-, lontophorese-, Neutral-, Stimulationselektroden).

Die vorgeschlagenen Elektroden werden diesen Anforderungen gerecht. Die Herstellung der Elektroden kann auf verschiedene Art und Weise erfolgen, wobei zu unterscheiden ist, ob eine Polymerbahn mit dem Leitlackmuster als Endlosdruck oder Einzeldruck bedruckt werden soll. Bei einem Einzeldruck werden Einzellabel gebildet, die entweder von einer Vorratsrolle oder von einem Stapelverbund aus in die Elektrodenfertigung integriert werden. Eine Verfahrenskombination besteht auch darin, daß die Bedruckung als Einzeldruck innerhalb der Fertigungslinie für die Elektroden integriert werden kann. Die Elektroden können als externen Anschluß bzw. Abgriffelemente wahlweise eine Druckknopfverbindung, eine Klemmverbindung oder eine angeschlagene Litze mit Sicherheitsstecker aufweisen.

Die erfindungsgemäß hergestellten Elektroden halten die durch den für EKG-Elektroden geltenden Standard ANSI/AAMI/EC12 vorgegebenen Grenzen für die elektrischen Kennwerte mühelos ein. So beträgt der Wert für die Impedanz eines Elektrodenpaares nur etwa 10% (ca. 200 Ohm) der Standardvorgabe, die Vorgaben für die Potentialabbaugeschwindigkeit nach Defibrillierungs-Stimulation oder für das Eigenpotential werden ebenso eingehalten.

Ein Vorteil dieser Elektrode besteht darin, daß die Artefaktneigung, das heißt die Abweichung der EKG-Kennlinie durch von außen aufgebrachte Bewegungen (z.B. Patienten- oder Kabelbewegungen) gegenüber einer marktgängigen Konstruktion mit Signalabgriff direkt über dem Leitgelkörper deutlich niedriger ist. Die Elektrode hat eine maximale Abweichung von der Normalen in der Größenordnung von etwa einem Drittel der Q-R-Zacke des EKG (höchster Ausschlag), während die unter gleichen praktischen Belastungen geprüfte konventionelle Elektrode Abweichungen bis zum Doppelten der Q-R-Zacke aufwies. Damit ist im ersten Fall das Signal beispielsweise einer automatischen Langzeitaufzeichnung noch voll zugängig, während im zweiten Fall eine Störphase ausgewiesen wird. Bei Patienten auftretende Unverträglichkeitsreaktionen der Haut bei Langzeitanwendungen können durch den ausreichend durchsichtigen Fensterbereich über dem transparenten Leitgelkörper auch optisch rechtzeitig wahrgenommen werden.

Der im Haftbereich zur Haut flexible Aufbau bewirkt ein angenehmes Tragegefühl beim Patienten (die Elektrode wird nicht als störend wahrgenommen) und eine lange Haftstabilität auf der Haut. Die Knickempfindlichkeit im Grenzbereich zwischen Abdeckfolie und Haftkleber zur Haut ist für eine übliche Belastung eines Dauer-EKG ausreichend reduziert, so daß es zu keinem Verlust der Langzeit-Signalgüte kommt.

Weitere Ausgestaltungsvarianten und deren Vorteile werden im Zusammenhang mit der nachstehenden Erläuterung der Erfindung erörtert. In der zugehörigen Zeichnung zeigen
- Fig. 1: eine Draufsicht auf den Bandlauf bei der Herstellung einer Elektrode mit einem Endlosdruck, als Halbschnitt,
- Fig. 2: die Draufsicht auf eine Einzelelektrode mit Endlosdruck,
- Fig. 3: einen Schnitt gemäß der Linie A-A in Fig. 2 in vergrößerter Darstellung,
- Fig. 4: die Draufsicht auf das Leitlackmuster eines Einzeldruckes,
- Fig. 5: die Draufsicht auf ein Einzellabel ohne Druckbild, mit den klebstoff-freien Bereichen,
- Fig. 6: die Draufsicht eines Einzellabels auf der Spendeunterlage einer antihaftbeschichteten Schutzbahn, vor dem Abspenden,
- Fig. 7: die Draufsicht auf die fertige Einzelelektrode mit Einzellabel gemäß Figur 6,
- Fig. 8: einen Schnitt gemäß der Linie A-A in Fig. 7,
- Fig. 9: die Draufsicht auf eine schematisch dargestellte Elektrode mit einer zusätzlichen, bedruckten, flexiblen Grifflaschenabdeckung als Verstärkung,
- Fig. 10: die Draufsicht auf ein vorbereitetes Einzellabel ohne Druckbild, dessen Außenkontur kleiner ist als die Außenkontur der Elektrode,
- Fig. 11: die Draufsicht eines Einzellabels auf der Spendeunterlage einer antihaftbeschichteten Schutzbahn, vor dem Abspenden,
- Fig. 12: die Draufsicht auf die fertige Elektrode gemäß Figur 11 und
- Fig. 13: einen Schnitt gemäß der Linie A-A in Figur 12 in vergrößerter Darstellung.

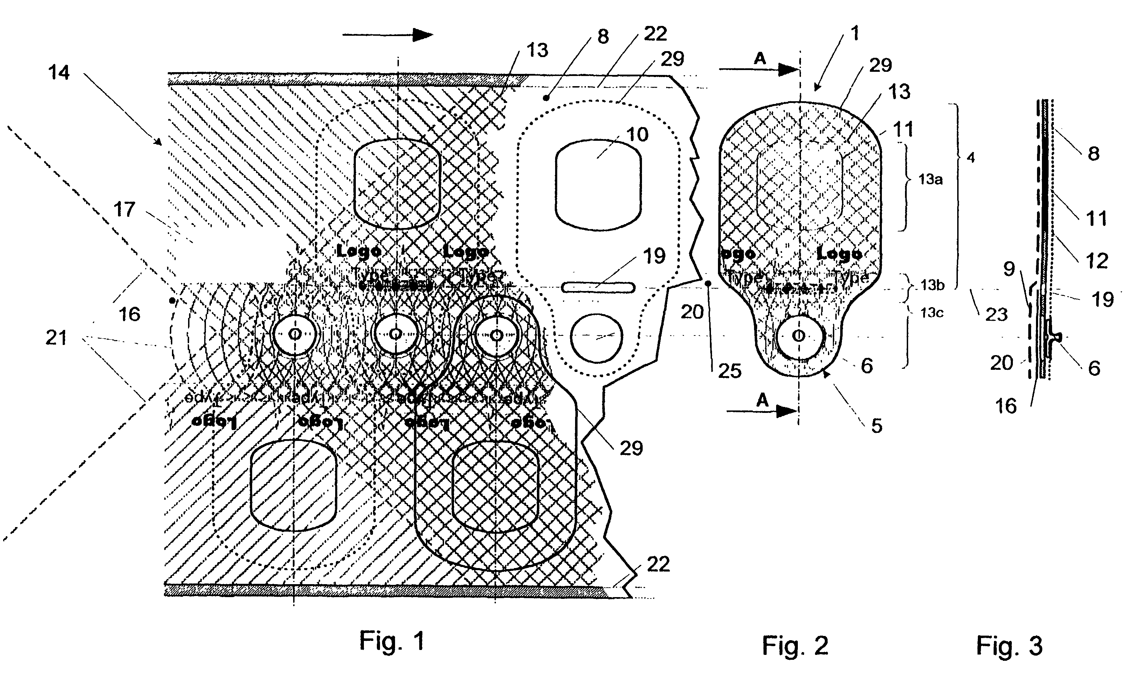

In den Figuren 2 und 3, 7 und 8 sowie 12 und 13 sind drei verschiedene Ausführungen von Elektroden dargestellt.

Die Elektroden 1, 2 und 3 besitzen im wesentlichen den gleichen Elektrodenaufbau und die gleiche Elektrodenform. Sie weisen einen auf der Haut des Patienten aufklebbaren Abschnitt 4 auf, in dem der Leitgelkörper 11 angeordnet ist. An einer der Schmalseiten dieses Abschnittes 4 befindet sich eine nach außen verjüngende Abgrifflasche 5, in der die erforderlichen Abgriffelemente oder Anschlußstellen für die Abgriffelemente vorgesehen sind. Geeignete Abgriffelemente sind z.B. eine Druckknopfverbindung 6, eine Klemmverbindung 7 oder eine Litze mit Verbindungselement, z.B. einem Sicherheitsstecker. An der zur Haut zeigenden Seite ist die Abgrifflasche 5 mittels einer nichtklebenden Abdeckung 16, z.B. einer Folie, versehen.

Jede dieser Elektroden 1, 2 und 3 besteht aus einer vorwiegend geschlossenzelligen Polymerschaumbahn 8 als Trägermaterial, deren mit der Hautoberfläche in Kontakt kommende Seite mit einem hautverträglichen Haftklebstoff beschichtet ist, die mit einer hautseitig nichthaftenden Elektrodenabdeckung 9 abgedeckt ist, die erst unmittelbar vor der Applikation der Elektrode entfernt wird. In dem Abschnitt 4 der Polymerschaumbahn 8 befindet sich eine Öffnung 10, in der der Leitgelkörper 11 angeordnet ist. Auf der hautabgewandten Seite der Polymerschaumbahn 8 ist eine Abdeckung aus einer flexiblen und reißfesten Polymerbahn 12 aufgeklebt. Die Polymerbahn 12 ist dabei ganzflächig, mit dem Abschnitt 4 und der sich daran anschließenden Abgrifflasche 5 verbunden und weist eine plane glatte Oberfläche auf. Der Leitgelkörper 11 bildet im wesentlichen mit der ihn umgebenden hautzugewandten, selbstklebenden Seite der Polymerschaumbahn 8 eine Planlage. Die Polymerbahn 12 ist auf der zur Polymerschaumbahn 8 zugewandten Seite mit einem Leitlackmuster 13 bedruckt, das die elektrische Verbindung zwischen dem Leitgelkörper 11 und der Abgriffstelle 6 oder 7 herstellt. Bei allen drei Elektroden 1, 2 und 3 ist die Polymerbahn 12 bereichsweise mit einem Leitlackmuster 13 unterschiedlicher Druckdichte bedruckt. In dem Bereich 13a, der sich über dem Leitgelkörper 11 befindet, ist die Druckdichte so bemessen, daß mindestens zwei Drittel dieses Bereiches 13a unbedruckt sind, so daß dieser Bereich noch eine ausreichende Transparenz aufweist, um durch den ebenfalls transparenten Leitgelkörper 11 eine visuelle Kontrolle der darunter befindlichen Haut beim Tragen der Elektrode zu ermöglichen. Bei den gezeigten Elektroden 1, 2 und 3 beträgt der unbedruckte Anteil in diesem Bereich 13a jeweils 85 %. In dem Bereich 13b der Elektroden, der den Übergang zur Abgrifflasche 5 bildet, ist die Druckdichte des Leitlackmuster 13 im Vergleich zu dem Leitlackmuster im Bereich 13a um mindestens ein Drittel höher. Infolge der höheren Druckdichte wird gewährleistet, daß auch bei starker Biegeund/oder Torsionsbeanspruchung dieses Bereiches 13b die erforderliche elektrische Leitfähigkeit nicht beeinträchtigt wird.

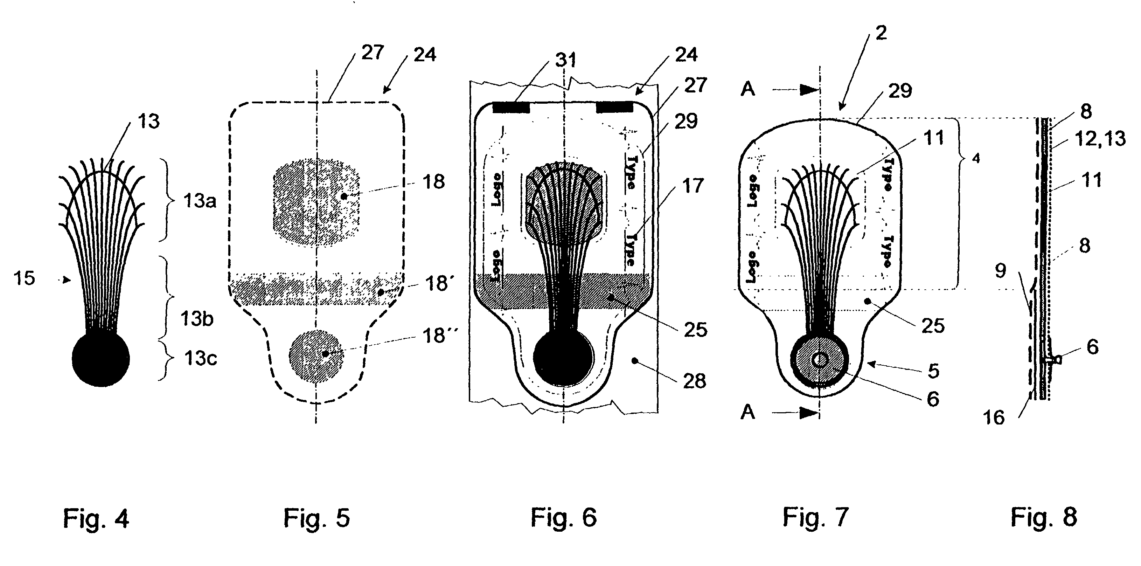

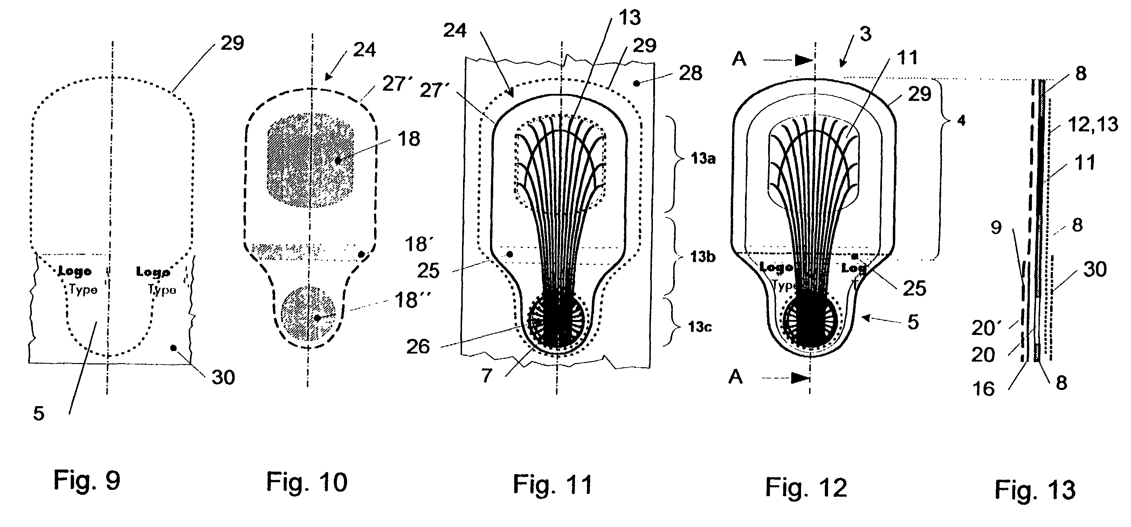

Im Bereich 13c der Abgrifflasche 5 kann die Druckdichte des Leitlackmusters 13 verringert werden, wobei sie jedoch mindestens so hoch sein sollte wie im Bereich 13a. Die Übergänge des Leitlackmusters zwischen den einzelnen Bereichen 13a bis 13c können fließend oder abgestuft (wie z.B. in Fig. 2) sein. Das entsprechende Leitlackmuster 13 kann entweder im Endlosdruck 14 (Figuren 1 bis 3) oder als Einzeldruck 15 auf die Polymerbahn 12 aufgedruckt werden. Zusätzlich, außerhalb des Bereiches 13a, kann die Polymerbahn 12 noch mit einem Logo 17, Schriftzug oder dgl. bedruckt sein. Die Elektroden 2 und 3 (Figuren 7 und 8 bzw. 12 und 13) sind mit einem Einzeldruck 15 versehen und im Bereich 25 der Knickstelle der Abgrifflasche 5 mit einer Haftungslücke 18' ausgestattet, d.h. im Knickbereich ist zwischen der Polymerschaumbahn 8 und der Polymerbahn 12 kein Klebstoff aufgetragen. Zusätzlich können im Knickbereich 25 der Polymerschaumbahn 8 Perforationen 19 in Form von Löchern oder kleinen Schlitzen eingebracht werden, um eine verbesserte Knickfähigkeit zu erreichen (Fig. 1). Die in den Figuren 12 und 13 gezeigte Elektrode 3 ist nicht mit einem Druckknopfanschluß ausgerüstet, sondern mit einem Anschluß für eine Klemmverbindung 7. Um dies zu ermöglichen, sind in der Abdeckung 16 der Abgrifflasche 5 und in der Polymerschaumbahn 8 deckungsgleiche Öffnungen 20, 20' eingebracht.

Weitere Einzelheiten der Elektroden 1, 2 und 3 werden im folgenden im Rahmen der Beschreibung zu den Herstellungsverfahren erläutert.

### A: Herstellung der Elektroden mit einem Leitlackmuster als Endlosdruck

Im ersten Verfahrensschritt wird eine transparente, flexible und reißfeste Polymerbahn 12, z.B. eine druckvorbehandelte Polyersterbahn von 20 bis 40 µm Stärke, mit einer Kennzeichnung 17 bedruckt. Diese im Endlosdruck, nach dem Siebdruckverfahren spiegelbildlich aufgebrachte Kennzeichnung 17 kann beispielsweise einzeln oder in Kombination Bezeichnungen wie ein bestimmtes Logo, zur Firma, Type usw. enthalten. Der Aufdruck der Kennzeichnung 17 erfolgt außerhalb des Bereiches 13a, der später den Leitgelkörper 11 abdeckt. Im nachfolgenden Verfahrensschritt wird im Siebdruckverfahren auf der in Richtung zu der Polymerschaumbahn 8 zeigenden Seite der Polymerbahn 12 das Leitlackmuster 13 als Endlosmuster 14 aufgedruckt. Dieses Leitlackmuster 13 wird in seiner Druckdichte variiert, nämlich in die Bereiche 13a, 13b und 13c, wie bereits erläutert.

In den Übergängen zwischen diesen Bereichen ist die Druckdichte jeweils abgestuft. Die erforderlichen Unterschiede in der Druckdichte werden durch Aneinanderreihung von Einzelmustem 21 erzielt. Die zur Abgrifflasche 5 hin höhere Druckdichte wird durch eine paarweise gegenüberliegende Anordnung der Einzelmuster 21 bewirkt, wie in Figur 1 gezeigt. Endlosdruck bzw. Endlosmuster bedeutet, daß keine differenzierte Musterzuordnung zur Einzelelektrode erfolgt, um beim späteren Verbund mit dem Trägermaterial, der Polymerschaumbahn 8, Probleme durch Musterversatz auszuschließen. Eine ständige Kontrolle der Druckparameter schon während des Druckvorganges erlaubt z.B. ein extra mitlaufender Kontrolldruck, der jedoch in der Zeichnung nicht dargestellt ist. Damit lassen sich Druckparameter, wie z.B. Auftragsstärke und Trocknung regeln, die später über Leitfähigkeit, Röntgentransparenz und andere Qualitätsparameter des Leitlackes entscheiden. Das Leitlackraster bzw. Leitlackdruckbild auf der Polymerbahn 12 erstreckt sich quer zu der mit einem Pfeil gekennzeichneten Bandlaufrichtung über die gesamte Bandbreite, die durch die mit der Position 22 gekennzeichneten Linien, definiert ist. Dadurch können evtl. Bandlaufungenauigkeiten während der Elektrodenfertigung im erforderlichen Maß toleriert werden. Wie Figur 1 zeigt, ist die Breite der Polymerschaumbahn 8 etwas größer als die der Polymerbahn 12. Für die Leitlackierung werden je nach Anwendungsfall mit entsprechenden Leitpartikeln versetzte Druckpasten, wie sie beispielsweise von der Leiterplattenfertigung her bekannt sind, eingesetzt. Besonders gut geeignet sind z.B. silberhaltige, teilweise mit Graphit versetzte, Zubereitungen. Für die besonderen Anforderungen der Ableitelektroden im EKG-Bereich werden diesen AgCI-Anteile zugesetzt. Die Abstimmung der Leitlackmuster (Dichteverteilung), Leitlackzusammensetzung und -druck (spezifischer Widerstand) erfolgt so, daß die für die EKG-Elektroden geltenden Mindestanforderungen des bereits erwähnten Standards erfüllt werden. Liegt die fertig bedruckte Polymerbahn 12 vor, kann der eigentliche Fertigungsprozeß in einem dafür geeigneten Automaten beginnen. Die Transportvorrichtung dieses Automaten kann taktweise oder im wesentlichen kontinuierlich arbeiten. Zunächst wird eine für solche Zwecke handelsübliche geschlossenzellige Polymerschaumbahn 8, welche beidseitig mit einem hautverträglichen Haftklebstoff versehen ist, in die Vorrichtung eingezogen. Um den Versteifungseffekt eines Sandwichaufbaus zu vermeiden, werden beidseitig haftklebstoffbeschichtete Bahnen von der Fabrikation her nur auf einer Seite mit einem beidseitig abweisenden Schutzfilm (z.B. silikonisiertem Papier) versehen. Eine Variante, dieses Material für Schneidwerkzeuge verarbeitbar zu gestalten, ist die zwischenzeitliche Abdeckung der beim Abwickeln von der Rolle freiwerdenden Haftseite mit einem entsprechenden lösbaren Schutzfilm. Wird die Polymerschaumbahn 8 an beiden Längsseiten mittels Klauen fixiert, muß, um ein Festkleben an den Klauen zu vermeiden, an beiden Rändern im Griffbereich die Haftklebstoffoberfläche abgedeckt bleiben. Zu gewährleisten ist, daß eine Haftklebstoffabdeckung auf der nach oben weisenden Seite bleibt, wenn, wie unten beschrieben, die Reaktionsmischung für den Leitgelkörper 11 eingebracht und polymerisiert wird. Nach dem Einbringen der Öffnungen 10 für den Leitgelkörper 11 und Öffnungen 20 für die Abgriffelemente 6 oder 7 als Signalanschluß mittels entsprechender Schnittwerkzeuge wird auf die klebende Seite der Polymerschaumbahn 8 die bedruckte Polymerbahn 12 mit der lackierten Seite zur Öffnung 10 zeigend aufgebracht. Zur Vermeidung des scharfen Knickens der bedruckten Polymerbahn 12, was auf die Dauer mit einer Beeinträchtigung der Leiterqualität verbunden wäre, wird im Knickbereich 25 der Abgrifflasche 5 eine Perforation 19 in Form von kleinen Löchern oder Schlitzen in die Polymerschaumbahn 8 eingebracht. Dadurch wird der Versteifungseffekt des Mehrschichtenaufbaus der Elektrode an dieser Stelle reduziert. Ist als Abgriffelement eine Druckknopfverbindung 7 für den externen Anschluß vorgesehen, kann es je nach Festigkeit der Polymerbahn 12 zweckmäßig sein, für die die Polymerbahn 12 durchdringende Nase des Druckknopfunterteils eine Öffnung in die Polymerbahn 12 einzubringen und anschließend die Druckknopfverbindung zu montieren. Das Unterteil der Druckknopfverbindung 7 steht dabei in direktem Kontakt mit dem zwischen Unter- und Oberteil eingeklemmten Leitlackmuster 13 und übernimmt somit den elektrischen Kontakt zwischen dem Leitlack und der Außenkontaktstelle der Elektrode, dem Oberteil der Druckknopfverbindung 7, dessen Anschlußnase bei Applikation der Elektrode 1 auf der Haut nach oben zeigt. Die hautzugewandte Seite der Polymerschaumbahn 8 wird im Bereich der Abgrifflasche 5 mittels einer flexiblen Isolierfolie 16 abgedeckt, deren Kante in Richtung zum Leitgelkörper 11 in den Figuren 2 und 3 mit dem Bezugszeichen 23 gekennzeichnet ist Die Abgrifflasche 5 ist somit gegen zufälligen Hautkontakt geschützt. Als Schutz gegen externes Verdrehen durch die vom Ableitkabel wirkenden Kräfte kann die Abgrifflasche 5 durch eine dickere Isolierfolie 16 versteift werden.

Im weiteren Verfahrensablauf kann nunmehr der Leitgelkörper 13 in die durch die Öffnung 10 in der Polymerschaumbahn 8 und deren Abdeckung mittels der mit Leitlack bedruckten Polymerbahn 12 gebildeten Kavität eingebracht werden.

Zweckmäßigerweise wird in die Kavität eine UV-härtbare Reaktionsmischung eingegeben, wie sie beispielsweise in DE 43 36 301 A1 beschrieben ist. Es besteht auch die Möglichkeit, beliebige andere Leitgelkörper, die diesen Zweck erfüllen, einzusetzen. Im Falle der Verwendung einer UV-härtbaren Mischung durchläuft das Band mit der eingefüllten Mischung einen UV-Tunnel zur Aushärtung der Reaktionsmischung, anschließend wird der Liner, welcher auf der nach oben gewandten Seite der Polymerschaumbahn 8 verblieben ist, abgezogen und ein durchgehender Liner 9, als Elektrodenabdeckung, der auch den Leitgelkörper 13 abdeckt, aufgebracht. Nun können die Elektroden 1 (einzeln oder auf Linerabschnitten) ausgeschnitten und verpackt werden.

### B: Herstellung der Elektroden ausgehend von einem Einzellabel mit einem Leitlackmuster als Einzeldruck

Die Verfahrensweise zur Herstellung der Elektroden 2, 3 mit einem Leitlackmuster als Einzeldruck wird im folgenden unter Bezugnahme auf die Figuren 4 bis 13 erläutert. Zuerst wird eine Polymerbahn 12, analog wie unter Beispiel A beschrieben, mit einem Logo 17 oder dgl. auf der der Haut zugewandten Seite bedruckt, wobei der Bereich, der den Leitgelkörper 11 abdeckt, unbedruckt bleibt. Für eine optische Ansteuerbarkeit beim Abspenden der Einzellabel wird im gleichen Druckvorgang eine Steuermarke 31 außerhalb der späteren Elektrodenkontur 29 gesetzt (Fig. 6). Danach wird das Leitlackmuster 13 mit den bereits erwähnten Bereichen 13a, 13b und 13c unterschiedlicher Druckdichte aufgetragen. Im Unterschied zu der Elektrode gemäß Fig. 1 bis 3, wird der Leitlack nur funktionsbedingt, als Einzeldruck 15, aufgetragen. D.h., die Leitlacklinien erstrecken sich nur im unmittelbaren Bereich zwischen dem Leitgelkörper 11 und der Abgriffstelle 6 oder 7 der Abgrifflasche 5. Im Bereich 13b werden die vom Bereich 13a kommenden Leitlackdrucklinien gebündelt, wodurch die erforderliche höhere Druckdichte gewährleistet wird. Im Bereich 13a des Leitgelkörpers 11 verlaufen die Leitlackdrucklinien auseinander, um die gewünschte Transparenz in diesem Bereich zu gewährleisten. Ein entsprechendes Druckbild eines Einzeldruck 15 ist in Fig. 4 gezeigt. Nach Fertigstellung der Druckbilder wird auf die mit Leitlack bedruckte Seite der Polymerbahn 12 im Siebdruckverfahren Haftklebstoff aufgetragen, wobei die den Leitgelkörper 11, den Knickbereich 25 und den Anschluß 26 bedeckenden Flächen frei von Klebstoff bleiben. Die dadurch bewirkten Haftungslücken 18, 18' und 18" sind in den Figuren 5 und 10 gezeigt. Die klebstoffbeschichtete Seite der Polymerbahn 12 wird nunmehr mit einer haftabweisenden, lösbaren Schutzbahn 28 (Fig. 6 bzw. Fig. 11) abgedeckt. Anschließend werden die Label 24 der Polymerbahn 12 mittels Messerschnitt entsprechend der vorgegebenen Außenkontur 27 vereinzelt und das Entfallmaterial abgezogen. Die Einzellabel 24 verbleiben auf der Endlosbahn der Schutzbahn 28 und werden als Rolle zur weiteren Verarbeitung bevorratet. Die Außenkontur 27 der in Fig. 5 und 6 gezeigten Label 24 ist dabei so bemessen, daß diese umlaufend die Außenkontur 29 der Elektroden bzw. des Trägermaterials um mindestens 1 mm überragt.

Im Gegensatz dazu ist die Außenkontur 27' der in den Figuren 10 und 11 gezeigten Einzellabel 24 kleiner als die Außenkontur 29 der Elektrode 3. Die Außenkontur 27' des Einzellabels 24 muß aber noch so groß sein, daß der Leitgelkörper 11 ausreichend abgedeckt ist und ein fester Verbund des Einzellabels 24 mit dem Trägermaterial 8 gewährleistet ist. Eine derartige Elektrode hat den Vorteil, daß im Randbereich eine maximale Flexibilität zur Haut hin besteht und die Elektrode so mechanisch besonders reizarm ist und einen guten Sitz auf der Haut gewährleistet.

Im Unterschied zu der im Verfahren A erläuterten Verfahrensweise ist die das Trägermaterial bildende Polymerschaumbahn 8 nur auf der hautzugewandten Seite mit einer Klebstoffbeschichtung zu versehen, da die aufzubringenden Einzellabel 24 bereits klebfähig ausgerüstet sind. Die Polymerschaumbahn 8 wird einem taktweise arbeitenden Fertigungsautomaten, wie bereits im Beispiel A erläutert, zugeführt. Ein zusätzlicher haftklebstoffabweisender Hilfsliner für die Stanzvorgänge in dem Fertigungsautomaten, wie im Beispiel A, ist nicht aufzubringen.

Der mit dem fertig einseitig haftklebstoffbeschichteten Trägermaterial 8 gelieferte Liner wird lediglich entlang der Haltekrallen der Transportkette geschlitzt, um nach dem Einbringen des Leitgelkörpers 11 ein Abheben des Liners zu ermöglichen. Im Verlauf des weiteren Bandtransportes werden analog wie im Beispiel A die Öffnungen 10 für den Leitgelkörper 13 und die Öffnungen 20 für den Anschluß der Abgriffelemente 6, 7 in die Polymerschaumbahn 8 eingebracht. Anschließend werden die Einzellabel 24 mittels eines handelsüblichen Labelspenders von der Vorratsrolle aus an die richtige Stelle auf der vorbereiteten Polymerschaumbahn 8 plaziert. Die Einzellabel 24 werden fest angedrückt, erforderlichenfalls kann dies unter Anwendung erhöhter Temperaturen erfolgen. Danach wird das Abgriffelement 6 oder 7 in der Abgrifflasche 5 montiert und der Leitgelkörper 11 in der bereits beschriebenen Art und Weise eingebracht. Im Anschluß daran werden der zur Polymerschaumbahn 8 gehörende Liner abgehoben, das bandförmige Abdeckmaterial 16 für die Unterseite der Abgrifflasche 5 aufgewalzt und der die Elektrodenunterseite abdeckende Liner 9. Abschließend werden die Elektroden entsprechend der vorgegebenen Außenkontur 29 ausgestanzt und verpackt.

Die Verfahrensweise, das Leitlackmuster als Einzeldruck 15 zu gestalten, hat im Vergleich zu der Verfahrensweise gemäß Beispiel A folgende Vorteile.

Das Leitlackmuster 13 kann, da es sich um einen klar zur Elektrodenkontur zugeordneten Druck handelt, den Erfordernissen besser angepaßt werden, als dies nach dem in Beispiel A beschriebenen Verfahren möglich ist. Allein durch den zugeordneten Druck lassen sich gegenüber der im Beispiel A genannten Verfahrensweise ca. 60% der hochwertigen Ag/AgCl-Lacke einsparen, bei sonst gleichem anwendungstechnischen Gebrauchswert. Die Mehrkosten des Druckauftrages auf die Einzellabel 24 werden durch die Einsparungen für den sonst erforderlichen doppelten Klebstoffauftrag auf dem Trägermaterial und den zweiten Hilfsliner wieder ausgeglichen. Die Elektrode ist optisch und funktionell variabel gestaltbar. Die vorgesehenen Maßnahmen zum Knickschutz für den Leitlack am Übergang zur Griffund Abgrifflasche garantieren die sichere Funktion der Elektrode auch unter extremen Belastungen über mehrere Tage Tragedauer.

Die Verfahrensweise zur Herstellung der Elektroden gemäß diesem Beispiel B kann auch dahingehend modifiziert werden, daß die Einzellabel 24 ohne Klebstoffauftrag an der Unterseite hergestellt werden. Die korrespondierende Seite des Trägermaterials, der Polymerschaumbahn 8, ist dann mit einem geeigneten Klebstoffauftrag zu versehen, wobei die erforderliche Haftungslücke im Knickbereich durch vorheriges Aufwalzen einer nichtklebenden flexiblen Streifenbahn auf die Polymerschaumbahn 8 gebildet wird, und dieser Bereich klebstofffrei bleibt. Die Einzellabel 24 werden dann von einem Stapel aus positioniert und mit den klebfähigen Flächen der Polymerschaumbahn verbunden.

### C: Herstellung der Elektroden ausgehend von einer zu bedruckenden Endlosbahn mit einem Leitlackmuster als Einzeldruck

Bei dieser Verfahrensweise wird eine Polymerbahn 12 als Endlosbahn eingesetzt, die erforderlichenfalls bereits mit einem Logo 17 bedruckt sein kann. Im Unterschied zum Beispiel A oder B wird das Leitlackmuster 13 auf diese Polymerbahn als Einzeldruck aufgebracht und der erforderliche Leitlackdruck erfolgt während der Fertigung der Elektroden in einem taktweise oder kontinuierlich arbeitenden Automaten. Die Aufbringung des Leitlackdruckes ist somit integrierter Bestandteil der Elektrodenfertigungslinie. In den Fertigungsautomaten werden zeitversetzt die transparente flexible Polymerbahn 12 und die Polymerschaumbahn 8 eingezogen. Die Zeitversetzung zu Produktionsbeginn ist erforderlich, da erst das Leitlackmuster aufgedruckt und getrocknet werden muß. Die Polymerbahn 12 wird dabei mit ihrer späteren Oberseite nach unten zeigend eingezogen und ist vollkommen klebstofffrei. Die zugeführte Polymerschaumbahn 8 ist analog beschaffen wie die Polymerschaumbahn gemäß dem Beispiel A. Die zeitversetzte Zwangszuführung der Polymerbahn 12 und der Polymerschaumbahn 8 in den Fertigungsautomaten gewährleistet die notwendige geometrische Zuordnung. Zuerst wird die Polymerbahn 12 mit den Leitlackmustern als Einzeldruck 15, z.B. mittels Tampondruck, bedruckt, wobei die einzelnen Leitlackmuster in Bandlaufrichtung in einem definierten Abstand zueinander aufgedruckt werden. Die Ausbildung der Leitlackmuster erfolgt dabei analog wie im Beispiel B. Die Polymerschaumbahn 8 wird oberhalb der Polymerbahn 12 zugeführt und deren klebfähige Seite mit einem lösbaren Schutzfilm abgedeckt. Die an beiden Seiten vorhandenen Schutzfilme werden im Fertigungsautomaten in Verarbeitungsrichtung geschlitzt. Der weitere Verfahrensablauf erfolgt dann analog wie im Beispiel A beschrieben. Die Integration der Leitlackdrucke innerhalb der Fertigungslinie ermöglicht einen einfachen und schnellen Wechsel bei notwendigen Sortimentsumstellungen, insbesondere auch hinsichtlich des Leitlackdruckbildes. Außerdem entfällt eine Zwischenlagerung von vorgefertigten Einzellabeln, wie dies bei einer Verfahrensweise gemäß Beispiel B erforderlich ist.

Die Verfahrensweise, das Leitlackmuster als Einzeldruck 15 zu gestalten, hat die gleichen Vorteile wie bereits im Beispiel B genannt. Als weiterer Vorteil kommt bei der Verfahrensweise gemäß dem Beispiel C noch die Möglichkeit einer variableren Gestalbarkeit hinzu.

Im folgenden werden noch einige Ausführungsvarianten für die gemäß den Beispielen A bis C hergestellten Elektroden 1,2 und 3 erläutert.

Anstelle der Druckknopfverbindung kann die Abgrifflasche 5 auch mit einem Anschluß für eine Klemmverbindung 7, z.B. einer Krokodilklemme, wie in den Figuren 12 und 13 gezeigt, ausgerüstet sein. In die Abdeckung 16 der Abgrifflasche 5 ist dann noch eine Öffnung 20' einzubringen, die nahezu deckungsgleich mit der Öffnung 20 der Polymerschaumbahn ist, um den notwendigen Kontakt zum Leitlackmuster 13 herzustellen. Diese Ausführung ist im Vergleich zu einer Druckknopfverbindung kostengünstiger und für Kurzzeitmessungen mit guter Signalqualität ausreichend.

Eine weitere Variante besteht darin, daß die Kavität für den Leitgelkörper und damit auch der Leitgelkörper größer ausgebildet werden als in der Zeichnung dargestellt, speziell für eine Aktivelektrode. Als Leitlack kann dann ein kostengünstiger Leitlack, z.B. auf Carbonbasis, verwendet werden und die Abgriffstelle an der Abgrifflasche 5 wird für einen Anschluß einer Litze mit einem Stiftstecker vorbereitet. Auf die Abgriffstelle bzw. den Anschluß 26 für ein Leitungssystem wird ein leitfähiger Klebstoff vor dem Aufbringen der Abdeckung für die Abgrifflasche aufgetragen und die Litze angeschlagen. Danach wird dann erst die Abdeckung 16 für die Abgrifflasche 5 aufgewalzt. Derartige Elektroden können z.B. im Bereich der Reizstrombehandlung eingesetzt werden. Infolge der Transparenz der Elektrode im Leitgelkörperbereich kann während der Behandlung erkannt werden, ob sich die applizierte Hautpartie durch zu hohe Strombelastungen rötet. Aus der Praxis ist bekannt, daß im Schmerzbild das Überlagern von Verbrennungsschmerz und "Kribbeln" der Stromeinwirkung für den Betroffenen nicht differenzierbar ist. Somit treten derartige Verbrennungen nicht selten auf und benötigen oft lange Zeit für ihre Abheilung.

Die bisher auf diesem Gebiet eingesetzten Elektroden ermöglichen keine Beobachtung der Hautpartie.

Die erfindungsgemäßen Elektroden können auch als röntgentransluzente Elektroden ausgebildet sein, wobei eine Leitlackzusammensetzung und eine Druckknopfverbindung eingesetzt werden, die röntgentransluzent sind. Damit steht für bestimmte Anwendungen eine Elektrode mit den o.g. Vorzügen zur Verfügung, die nicht oder nur unwesentlich im Röntgenbild eines applizierten Patienten sichtbar und damit störend ist.

Der Leitgelkörper 13 kann auch aus optischen Gründen schwach eingefärbt sein, ohne dadurch die Transparenz zu beeinträchtigen.

Bei einer weiteren Elektrodenausführung ist die Oberseite der Grifflasche 5, mit einer dauerhaft aufgebrachten Verstärkungsfolie 30 verbunden (Fig. 9), insbesondere wenn als externer Anschluß eine Klemmverbindung 7 vorgesehen ist. Die Verstärkungsfolie 30 sollte auch bedruckbar sein. Eine derartige Elektrode 3 ist beständiger gegen eine mechanische Zerstörung des Abgriffbereiches, und der Abgriffbereich kann zusätzlich noch mit einer Kennzeichnung markiert werden.

## Patentansprüche

1. Biomedizinische Elektrode zur Übertragung elektrischer Signale von der Haut zu einem elektromedizinischen Gerät oder zur Übertragung externer Signale zur Haut, bestehend aus einer polymeren Schaumstoffbahn (8) als Trägermaterial, deren mit der Hautoberfläche in Kontakt kommende Seite mit einem hautverträglichen Haftklebstoff beschichtet und mit einer entfernbaren Abdeckung (9) abgedeckt ist, in die mindestens eine Öffnung (10) für einen Leitgelkörper (11) eingebracht ist, auf deren hautabgewandter Seite eine Abdeckung aus einer optisch transparenten, flexiblen und reißfesten Polymerbahn (12) aufgeklebt ist, wobei der Leitgelkörper (11) im wesentlichen mit der ihn umgebenden hautzugewandten, selbstklebenden Seite der Schaumstoffbahn (8) eine Planlage bildet und mittels einem auf der der Schaumstoffbahn (8) zugewandten Seite der Polymerbahn (12) aufgedruckten Leitlack mit einem außerhalb der Gelkörperöffnung (10) gelegenen Anschluß (26) für ein Leitungssystem verbunden ist, **dadurch gekennzeichnet, daß** die Polymerbahn (12) ganzflächig mit dem Trägermaterial der Schaumstoffbahn (8) verbunden ist und eine plane, glatte Oberfläche aufweist, die Schaumstoffbahn (8) eine sich an ihrem äußeren Rand anschließende flexible Abgrifflasche (5) aufweist, in der der Anschluß (26) angeordnet ist, und die an der zur Haut zeigenden Seite mit einer nichtklebenden Abdeckung (16) ausgestattet ist, und die Polymerbahn (12) mindestens im Bereich des Leitgelkörpers (11) und im Bereich vom Leitgelkörper (11) bis zu dem Anschluß (26) mit einem Leitlackmuster (13) unterschiedlicher Druckdichte versehen ist, wobei die Druckdichte des Leitlackmusters (13) im Bereich (13a) des Leitgelkörpers (11) so bemessen ist, daß mindestens zwei Drittel dieses Bereiches optisch transparent sind, in dem Bereich (13b), der den Übergang vom Leitgelkörper (11) zu der Abgrifflasche (5) bildet, die Druckdichte des Leitlackmusters (13) gegenüber dem Bereich (13a) des Leitgelkörpers (11) um mindestens ein Drittel, bezogen auf die mit Leitlack bedruckte Fläche in diesem Bereich, erhöht ist, und die Druckdichte im Bereich (13c), der sich unmittelbar um den in der Abgrifflasche (5) angeordneten Anschluß befindet, mindestens so hoch ist wie die Druckdichte im Bereich (13a) des Leitgelkörpers (11).

2. Elektrode nach Anspruch 1, **dadurch gekennzeichnet, daß** im Bereich (25) der Knickstelle der Abgrifflasche (5) zum übrigen Elektrodenkörper zwischen der Polymerschaumbahn (8) und der aufgeklebten Polymerfolie (12) eine Haftungslücke (18') angeordnet ist und/oder die Polymerschaumbahn perforiert ist.

3. Elektrode nach einem der Ansprüche 1 oder 2, **dadurch gekennzeichnet, daß** die hautseitige, lösbare, nichthaftende Abdeckung (9), die unmittelbar vor der Applikation entfernt wird, mindestens den außerhalb der Grifflaschenabdeckung (16) liegenden Abschnitt (4) vollständig abdeckt und an ihrer haftklebstoffzugewandten Seite mit einer Antihaftbeschichtung versehen ist.

4. Elektrode nach einem der Ansprüche 1 bis 3, **dadurch gekennzeichnet, daß** auf der hautabgewandten Seite mindestens im Bereich der Abgrifflasche (5) eine zusätzliche flexible Bahn (30) aufgebracht ist, die mindestens in den Bereich (25) der Knickstelle ragt.

5. Elektrode nach einem der Ansprüche 1 bis 4, **dadurch gekennzeichnet, daß** die auf der Polymerschaumbahn (8) aufgeklebte bedruckte Polymerbahn (12) eine größere Außenkontur (27) aufweist, als die Außenkontur (29) des Trägermaterials.

6. Elektrode nach einem der Ansprüche 1 bis 5, **dadurch gekennzeichnet, daß** die auf der Polymerschaumbahn (8) aufgeklebte bedruckte Polymerbahn (12) eine kleinere Außenkontur (27') aufweist als die Außenkontur (29) des Trägermaterials, wobei die Polymerbahn (12) mindestens den Leitgelkörper (11) allseitig überragt und sich bis zum Abgriffelement (6, 7) erstreckt.

7. Elektrode nach einem der Ansprüche 1 bis 6, **dadurch gekennzeichnet, daß** der Anschluß (26) in der Abgrifflasche mit einer Druckknopfverbindung (6) oder einer Klemmverbindung (7) oder einer Litze mit einem Verbindungselement verbunden ist.

8. Verfahren zur Herstellung biomedizinischer Elektroden, insbesondere nach einem der Ansprüche 1 bis 7, durch folgende Verfahrensschritte:
a) Bedrucken einer optisch transparenten, flexiblen und reißfesten Polymerbahn (12) mit einem Leitlackmuster (13) unterschiedlicher Druckdichte als Endlosdruck (14), wobei die Druckdichte durch Aneinanderreihung von Einzelmustern (21) variiert wird und das so hergestellte Leitlackmuster (13) für jede Elektrode im Bereich (13a) des Leitgelkörpers (11) in seiner Druckdichte so eingestellt wird, daß mindestens zwei Drittel dieses Bereiches (13a) optisch transparent ausgebildet werden, der Bereich (13b) der Polymerbahn (12), der den Übergang zur Abgrifflasche (5) bildet, mit einem Leitlackmuster (13) höherer Druckdichte versehen wird, die um mindestens ein Drittel höher ist als die Druckdichte im Bereich (13a) des Leitgelkörpers (11), und das Leitlackmuster (13) im Bereich (13c), der sich unmittelbar um das in der Abgrifflasche (5) angeordnete Abgriffelement (6, 7) befindet, mit einer Druckdichte ausgebildet wird, die mindestens so hoch ist wie die Druckdichte im Bereich (13a) des Leitgelkörpers (11),
b) einem taktweise oder kontinuierlich arbeitenden Fertigungsautomaten wird eine beidseitig mit Haftklebstoff beschichtete, vorwiegend geschlossenzellige Polymerschaumbahn (8) zugeführt, wobei mindestens deren hautzugewandte Seite hautverträglich ausgerüstet ist, und eine Seite mit einem lösbaren Schutzfilm versehen ist und die andere, freie, klebfähige Seite mit einem lösbaren Schutzfilm abgedeckt wird, und die Schutzfilme aus fertigungstechnischen Gründen in Verarbeitungsrichtung geschlitzt werden,
c) in die beidseitig nichtklebend abgedeckte Polymerschaumbahn (8) wird mindestens eine Öffnung (10) für den Leitgelkörper (11) eingebracht,
d) der hautabgewandte, im Fertigungsautomat nach unten zeigende Schutzfilm wird außerhalb der Befestigungselemente des Automaten abgehoben,
e) auf die nunmehr klebfähige Fläche der Polymerschaumbahn (8) wird die gemäß dem Verfahrensschritt a) hergestellte Polymerbahn (12) mit der lackierten Seite nach oben weisend, aufgebracht,
f) danach werden die Abgriffelemente (6, 7) für den externen Anschluß montiert,
g) in die durch die Öffnung (10) in der Polymerschaumbahn (8) und die von unten aufgeklebte Polymerbahn (12) gebildete Kavität wird ein optisch transparenter Leitgelkörper (11) mit der Oberseite der Polymerschaumbahn (8) plan abschließend eingebracht,
h) anschließend wird der nach oben weisende, außerhalb der Befestigungselemente des Automaten befindliche Schutzfilm von der Polymerschaumbahn (8) abgezogen, und der hautzugewandte Teil, der die Abgrifflasche (5) bildet, mit einer elektrisch isolierenden, flexiblen und schweißresistenten Abdeckung (16) versehen,
i) danach werden mindestens die noch klebenden Flächen, einschließlich die des Leitgelkörpers (11) an der hautzugewandten Seite mit einem lösbaren Schutzfilm (9) abgedeckt und
j) abschließend die fertige Elektrode (1) ausgestanzt.

9. Verfahren zur Herstellung biomedizinischer Elektroden, insbesondere nach einem der Ansprüche 1 bis 7, durch folgende Verfahrensschritte:
a) Bedrucken einer optisch transparenten, flexiblen und reißfesten Polymerbahn (12) mit einem Leitlackmuster (13) unterschiedlicher Druckdichte als Einzeldruck (15), wobei das Leitlackmuster (13) für jede Elektrode im Bereich (13a) des Leitgelkörpers (11) in seiner Druckdichte so eingestellt wird, daß mindestens zwei Drittel dieses Bereiches (13a) optisch transparent ausgebildet werden, der Bereich (13b) der Polymerbahn (12), der den Übergang zur Abgrifflasche (5) bildet, mit einem Leitlackmuster (13) höherer Druckdichte versehen wird, die um mindestens ein Drittel höher ist als die Druckdichte im Bereich (13a) des Leitgelkörpers (11), und das Leitlackmuster (13) im Bereich (13c), der sich unmittelbar um das in der Abgrifflasche (5) angeordnete Abgriffelement (6, 7) befindet, mit einer Druckdichte ausgebildet wird, die mindestens so hoch ist wie die Druckdichte im Bereich (13a) des Leitgelkörpers (11), die unmittelbaren Berührungsflächen der Polymerbahn (12) mit der Polymerschaumbahn (8) als Trägermaterial mit einem Haftklebstoff versehen werden und die haftklebstoffbeschichtete Seite der Polymerbahn (12) mit einer Schutzbahn (28) abgedeckt wird und aus der Polymerbahn (12) fertige, zwischenlagerungsfähige, leitfähige Einzellabel (24) gebildet werden,
b) in einen Fertigungsautomaten eine das Trägermaterial bildende, vorwiegend geschlossenzellige Polymerschaumbahn (8), die nur an der der Haut zugewandten Seite mit einem Haftklebstoff beschichtet ist, eingezogen wird, wobei diese Seite mit einem Schutzfilm abgedeckt ist und nach oben zeigt, und der Schutzfilm entlang der Halteelemente des Automaten geschlitzt wird,
c) in die Polymerschaumbahn (8) wird mindestens eine Öffnung (10) für den Leitgelkörper (11) eingebracht,
d) ein fertiges Einzellabel (24) wird, wie gemäß Verfahrensschritt a) vorgefertigt, bereitgestellt und in die richtige Position unterhalb der Unterseite der Polymerschaumbahn (8) gebracht und mittels Andruckelementen mit der Polymerschaumbahn (8) dauerhaft verklebt, wobei das Leitlackmuster (13) in Richtung der eingebrachten Öffnung (10) für den Leitgelkörper (11) nach oben zeigt,
e) in die durch die Öffnung (10) in der Polymerschaumbahn (8) und das aufgeklebte Einzellabel (24) gebildete Kavität wird ein optisch transparenter Leitgelkörper (11) mit der Oberseite der Polymerschaumbahn (8) plan abschließend eingebracht,
f) anschließend werden die Abgriffelemente (6, 7) für den externen Anschluß montiert,
g) danach wird der zum Trägermaterial (8) gehörende Schutzfilm abgezogen, ein bandförmiges, hautseitig nichtklebendes, flexibles, elektrisch isolierendes und schweißresistentes Abdeckmaterial (16) an der Unterseite der Abgrifflasche (5) aufgewalzt, und ein die Elektrodenunterseite abdeckender Schutzfilm (9) aufgebracht, der den Leitgelkörper (11) und die Haftklebschicht des Trägermaterials (8) abdeckt, und
h) abschließend die fertige Elektrode (2, 3) ausgestanzt.

10. Verfahren zur Herstellung biomedizinischer Elektroden, insbesondere nach einem der Ansprüche 1 bis 7, durch folgende Verfahrensschritte:
a) in einen taktweise oder kontinuierlich arbeitenden Fertigungsautomaten werden zeitversetzt, zuerst eine optisch transparente, flexible und reißfeste Polymerbahn (12) und danach eine das Trägermaterial bildende, vorwiegend geschlossenzellige Polymerschaumbahn (8) eingezogen, wobei die Polymerschaumbahn (8) beidseitig mit Klebstoff beschichtet ist und mindestens die hautzugewandte Seite hautverträglich ausgerüstet ist, und eine Seite mit einem lösbaren Schutzfilm versehen ist und die andere, freie, klebfähige Seite mit einem lösbaren Schutzfilm abgedeckt wird, und die Schutzfilme aus fertigungstechnischen Gründen in Verarbeitungsrichtung geschlitzt werden,
b) in dem Fertigungsautomaten die Polymerbahn (12) mit einem Leitlackmuster (13) unterschiedlicher Druckdichte als Einzeldruck (15) bedruckt wird, wobei das Leitlackmuster (13) für jede Elektrode im Bereich (13a) des Leitgelkörpers (11) in seiner Druckdichte so eingestellt wird, daß mindestens zwei Drittel dieses Bereiches (13a) optisch transparent ausgebildet werden, der Bereich (13b) der Polymerbahn (12), der den Übergang zur Abgrifflasche (5) bildet, mit einem Leitlackmuster (13) höherer Druckdichte versehen wird, die um mindestens ein Drittel höher ist als die Druckdichte im Bereich (13a) des Leitgelkörpers (11), und das Leitlackmuster (13) im Bereich (13c), der sich unmittelbar um das in der Abgrifflasche (5) angeordnete Abgriffelement (6, 7) befindet, mit einer Druckdichte ausgebildet wird, die mindestens so hoch ist wie die Druckdichte im Bereich (13a) des Leitgelkörpers (11), und die jeweiligen Leitlackmuster (13) entsprechend der Elektrodenzuordnung in einem definierten Abstand zueinander angeordnet werden,
c) in die beidseitig nichtklebend abgedeckte Polymerschaumbahn (8) wird mindestens eine Öffnung (10) für den Leitgelkörper (11) eingebracht,
d) der hautabgewandte, im Fertigungsautomat nach unten zeigende Schutzfilm wird außerhalb der Befestigungselemente des Automaten abgehoben,
e) auf die nunmehr klebfähige Fläche der Polymerschaumbahn (8) wird die Polymerbahn (12) mit der lackierten Seite nach oben weisend, entsprechend der Elektrodenzuordnung aufgebracht,
f) danach werden die Abgriffelemente (6, 7) für den externen Anschluß montiert,
g) in die durch die Öffnung (10) in der Polymerschaumbahn (8) und die von unten aufgeklebte Polymerbahn (12) gebildete Kavität wird ein optisch transparenter Leitgelkörper (11) mit der Oberseite der Polymerschaumbahn (8) plan abschließend eingebracht,
h) anschließend wird der nach oben weisende, außerhalb der Befestigungselemente des Automaten befindliche Schutzfilm abgezogen, der hautzugewandte Teil der Abgrifflasche (5) mit einer nichtklebenden, elektrisch isolierenden, flexiblen und schweißresistenten Abdeckung (16) versehen,
i) danach werden mindestens die noch klebenden Flächen, einschließlich die des Leitgelkörpers (11) an der hautzugewandten Seite mit einem lösbaren Schutzfilm (9) abgedeckt und
j) abschließend die fertige Elektrode (2, 3) ausgestanzt.

11. Elektrode nach mindestens einem der vorhergehenden Ansprüche, **dadurch gekennzeichnet, daß** diese aus röntgentransluzentem Material besteht.

## Claims

1. Biomedical electrode for the transmission of electrical signals from the skin to an electromedical device or for the transmission of external signals to the skin, comprising a polymeric foam sheet (8) as a supporting material, the side of which that comes into contact with the surface of the skin being coated with a skin-compatible contact adhesive and covered with a removable covering (9), in which there has been made at least one opening (10) for a conductive gel body (11), the side of which that is remote from the skin having a covering comprising an optically transparent, flexible and tear-resistant polymer sheet (12) adhesively attached on it, the conductive gel body (11) essentially forming a planar layer with the skin-facing self-adhesive side of the foam sheet (8) surrounding it and being connected by means of a conductive coating printed onto the side of the polymer sheet (12) facing the foam sheet (8) to a terminal (26), situated outside the gel body opening (10), for a system of lines, **characterized in that** the polymer sheet (12) is bonded over its full surface area to the supporting material of the foam sheet (8) and has a planar, smooth surface, the foam sheet (8) has a flexible gripping tab (5), which adjoins its outer edge and in which the terminal (26) is arranged, and is provided on the side facing the skin with a non-adhesive covering (16), and the polymer sheet (12) is provided at least in the region of the conductive gel body (11) and in the region from the conductive gel body (11) up to the terminal (26) with a pattern of conductive coating (13) of varying print density, the print density of the pattern of conductive coating (13) in the region (13a) of the conductive gel body (11) being set at such a level that at least two thirds of this region are optically transparent, the print density of the pattern of conductive coating (13) in the region (13b) which forms the transition from the conductive gel body (11) to the gripping tab (5) being increased in comparison with the region (13a) of the conductive gel body (11) by at least one third, based on the surface area printed with conductive coating in this region, and the print density in the region (13c) which is located directly around the terminal arranged in the gripping tab (5) being at least as high as the print density in the region (13a) of the conductive gel body (11).

2. Electrode according to Claim 1, **characterized in that**, in the region (25) of the bending point of the gripping tab (5) with respect to the remaining electrode body, an adhesion gap (18') is arranged between the polymer foam sheet (8) and the adhesively attached polymer film (12) and/or the polymer foam sheet is perforated.

3. Electrode according to either of Claims 1 and 2, **characterized in that** the skin-side detachable, non-adhering covering (9), which is removed immediately before application, completely covers at least the portion (4) lying outside the gripping tab covering (16) and is provided with a non-stick coating on its side facing the contact adhesive.

4. Electrode according to one of Claims 1 to 3, **characterized in that**, on the side remote from the skin, at least in the region of the gripping tab (5), an additional flexible sheet (30), which protrudes at least into the region (25) of the bending point, is applied.

5. Electrode according to one of Claims 1 to 4, **characterized in that** the printed polymer sheet (12) adhesively attached to the polymer foam sheet (8) has a greater outer contour (27) than the outer contour (29) of the supporting material.

6. Electrode according to one of Claims 1 to 5, **characterized in that** the printed polymer sheet (12) adhesively attached to the polymer foam sheet (8) has a smaller outer contour (27') than the outer contour (29) of the supporting material, the polymer sheet (12) at least reaching over the conductive gel body (11) on all sides and extending as far as the gripping element (6, 7).

7. Electrode according to one of Claims 1 to 6, **characterized in that** the terminal (26) in the gripping tab is connected to a connecting element by a press-stud connection (6) or a clamping connection (7) or a stranded wire.

8. Method of producing biomedical electrodes, in particular according to one of Claims 1 to 7, by the following method steps:
a) printing onto an optically transparent, flexible and tear-resistant polymer sheet (12) a pattern of conductive coating (13) of varying print density as an endless print (14), the print density being varied by arranging individual patterns (21) in series with one another and the pattern of conductive coating (13) produced in this way for each electrode in the region (13a) of the conductive gel body (11) being set in its print density such that at least two thirds of this region (13a) are made optically transparent, the region (13b) of the polymer sheet (12) which forms the transition to the gripping tab (5) being provided with a pattern of conductive coating (13) of a higher print density, which is at least one third higher than the print density in the region (13a) of the conductive gel body (11), and the pattern of conductive coating (13) in the region (13c) which is directly around the gripping element (6, 7) arranged in the gripping tab (5) being formed with a print density which is at least as high as the print density in the region (13a) of the conductive gel body (11),
b) a cyclically or continuously operating automatic production unit is fed a predominantly closed-cell polymer foam sheet (6), coated on both sides with contact adhesive, at least the skin-facing side of which sheet being provided with a skin-compatible finish, and one side being provided with a detachable protective film and the other, free, adhesive side being covered with a detachable protective film, and the protective films being slit in the processing direction for technical production-related reasons,
c) at least one opening (10) for the conductive gel body (11) is made in the polymer foam sheet (8) covered non-adhesively on both sides,
d) the skin-remote protective film, facing downwards in the automatic production unit, is lifted off outside the fastening elements of the automatic unit,
e) the polymer sheet (12) prepared according to method step a) is applied with the coated side facing upwards to the now adhesive surface of the polymer foam sheet (8),
f) after that, the gripping elements (6, 7) for the external terminal are fitted,
g) an optically transparent conductive gel body (11) is introduced into the cavity formed by the opening (10) in the polymer foam sheet (8) and the polymer sheet (12) adhesively attached from below, the said body terminating flat with the upper side of the polymer foam sheet (8),
h) subsequently, the upwardly facing protective film, located outside the fastening elements of the automatic unit, is pulled off from the polymer foam sheet (8), and the skin-facing part, which forms the gripping tab (5), is provided with an electrically insulating, flexible and perspiration-resistant covering (16),
i) after that, at least the still adhering surfaces, including that of the conductive gel body (11), on the skin-facing side are covered with a detachable protective film (9) and
j) finally, the finished electrode (1) is punched out.

9. Method of producing biomedical electrodes, in particular according to one of Claims 1 to 7, by the following method steps:
a) printing onto an optically transparent, flexible and tear-resistant polymer sheet (12) a pattern of conductive coating (13) of varying print density as an individual print (15), the pattern of conductive coating (13) for each electrode in the region (13a) of the conductive gel body (11) being set in its print density such that at least two thirds of this region (13a) are made optically transparent, the region (13b) of the polymer sheet (12) which forms the transition to the gripping tab (5) being provided with a pattern of conductive coating (13) of a higher print density, which is at least one third higher than the print density in the region (13a) of the conductive gel body (11), and the pattern of conductive coating (13) in the region (13c) which is directly around the gripping element (6, 7) arranged in the gripping tab (5) being formed with a print density which is at least as high as the print density in the region (13a) of the conductive gel body (11), the surfaces of the polymer sheet (12) in direct contact with the polymer foam sheet (8) as the supporting material being provided with a contact adhesive and the contact-adhesive-coated side of the polymer sheet (12) being covered with a protective sheet (28), and finished conductive individual labels (24) suitable for intermediate storage being formed from the polymer sheet (12),
b) an automatic production unit has drawn into it a predominantly closed-cell polymer foam sheet (8), which forms the supporting material and is coated with a contact adhesive only on the side facing the skin, this side being covered with a protective film and facing upwards, and the protective film is slit along the holding elements of the automatic unit,
c) at least one opening (10) for the conductive gel body (11) is made in the polymer foam sheet (8),
d) a finished individual label (24), prefabricated according to method step a), is created and brought into the correct position under the underside of the polymer foam sheet (8) and is permanently adhesively bonded to the polymer foam sheet (8) by means of pressure-applying elements, the pattern of conductive coating (13) facing upwards in the direction of the opening (10) made for the conductive gel body (11),
e) an optically transparent conductive gel body (11) is introduced into the cavity formed by the opening (10) in the polymer foam sheet (8) and the adhesively attached individual label (24), the said body terminating flat with the upper side of the polymer foam sheet (8),
f) subsequently, the gripping elements (6, 7) for the external terminal are fitted,
g) after that, the protective film belonging to the supporting material (8) is pulled off, a strip-shaped flexible, electrically insulating and perspiration-resistant covering (16), not adhering on the skin side, is rolled onto the underside of the gripping tab (5), and a protective film (9), covering the underside of the electrode, is applied, covering the conductive gel body (11) and the contact adhesive layer of the supporting material (8), and
h) finally, the finished electrode (2, 3) is punched out.

10. Method of producing biomedical electrodes, in particular according to one of Claims 1 to 7, by the following method steps:
a) a cyclically or continuously operating automatic production unit has drawn into it, at different times, firstly an optically transparent, flexible and tear-resistant polymer sheet (12) and after that a . predominantly closed-cell polymer foam sheet (8), forming the supporting material, the polymer foam sheet (8) being coated on both sides with adhesive and at least the skin-facing side being provided with a skin-compatible finish, and one side being provided with a detachable protective film and the other, free, adhesive side being covered with a detachable protective film, and the protective films being slit in the processing direction for technical production-related reasons,
b) in the automatic production unit, the polymer sheet (12) has printed onto it a pattern of conductive coating (13) of varying print density as an individual print (15), the pattern of conductive coating (13) for each electrode in the region (13a) of the conductive gel body (11) being set in its print density such that at least two thirds of this region (13a) are made optically transparent, the region (13b) of the polymer sheet (12) which forms the transition to the gripping tab (5) being provided with a pattern of conductive coating (13) of a higher print density, which is at least one third higher than the print density in the region (13a) of the conductive gel body (11), and the pattern of conductive coating (13) in the region (13c) which is directly around the gripping element (6, 7) arranged in the gripping tab (5) being formed with a print density which is at least as high as the print density in the region (13a) of the conductive gel body (11), and the respective patterns of conductive coating (13) being arranged at a defined distance in relation to one another in a way corresponding to the electrode assignment,
c) at least one opening (10) for the conductive gel body (11) is made in the polymer foam sheet (8) covered non-adhesively on both sides,
d) the skin-remote protective film, facing downwards in the automatic production unit, is lifted off outside the fastening elements of the automatic unit,
e) the polymer sheet (12) is applied with the coated side facing upwards, in a way corresponding to the electrode assignment, to the now adhesive surface of the polymer foam sheet (8),
f) after that, the gripping elements (6, 7) for the external terminal are fitted,
g) an optically transparent conductive gel body (11) is introduced into the cavity formed by the opening (10) in the polymer foam sheet (8) and the polymer sheet (12) adhesively attached from below, the said body terminating flat with the upper side of the polymer foam sheet (8),
h) subsequently, the upwardly facing protective film, located outside the fastening elements of the automatic unit, is pulled off, and the skin-facing part of the gripping tab (5) is provided with a non-adhering, electrically insulating, flexible and perspiration-resistant covering (16),
i) after that, at least the still adhering surfaces, including that of the conductive gel body (11), on the skin-facing side are covered with a detachable protective film (9) and
j) finally, the finished electrode (1) is punched out.

11. Electrode according to at least one of the preceding claims, **characterized in that** it consists of X-ray-translucent material.

## Revendications

1. Electrode biomédicale pour le transfert de signaux électriques de la peau à un appareil électromédical ou pour le transfert de signaux externes à la peau, constituée d'une bande de mousse polymère (8) comme matériau support, dont la face venant en contact avec la surface de la peau est revêtue d'une colle de contact compatible avec la peau et est recouverte d'un recouvrement amovible (9), dans laquelle est ménagée au moins une ouverture (10) pour un corps de gel conducteur (11), sur la face opposée à la peau de laquelle est collé un recouvrement formé d'une bande polymère (12) optiquement transparente, souple et résistant à la déchirure, le corps de gel conducteur (11) formant sensiblement avec la face autocollante de la bande de mousse (8) tournée vers la peau et l'entourant une couche plane et étant reliée, au moyen d'un vernis conducteur imprimé sur la face de la bande polymère (12) tournée vers la bande de mousse (8), à une connexion (26) située à l'extérieur de l'ouverture (10) du corps de gel pour un système de conduction, **caractérisée en ce que** la bande polymère (12) est reliée, sur toute sa surface, au matériau support de la bande de mousse (8) et présente une surface plane lisse, la bande de mousse (8) présente une languette de prise (5) souple se raccordant sur son bord externe, languette dans laquelle la connexion (26) est disposée, et la face tournée vers la peau est munie d'un recouvrement non collant (16), et la bande polymère (12) est pourvue, au moins dans la zone du corps de gel conducteur (11) et dans la zone du corps de gel conducteur (11) jusqu'à la connexion (26), d'un motif de vernis conducteur (13) de différente densité d'impression, la densité d'impression du motif de vernis conducteur (13) étant mesurée dans la zone (13a) du corps de gel conducteur (11) de sorte qu'au moins deux tiers de cette zone soient optiquement transparents, que, dans la zone (13b), qui forme la transition du corps de gel conducteur (11) à la languette de prise (5), la densité d'impression du motif de vernis conducteur (13) soit augmentée par rapport à la zone (13a) du corps de gel conducteur (11) d'au moins un tiers par rapport à la surface imprimée de vernis conducteur dans cette zone, et que la densité d'impression dans la zone (13c), qui se trouve directement autour de la connexion agencée dans la languette de prise (5), soit au moins aussi élevée que la densité d'impression dans la zone (13a) du corps de gel conducteur (11).

2. Electrode selon la revendication 1, **caractérisée en ce que**, dans la zone (25) du point de pliage de la languette de prise (5) par rapport au reste du corps de l'électrode, est ménagée entre la bande de mousse polymère (8) et la feuille de polymère collée dessus (12) une discontinuité d'adhérence (18') et/ou la bande de mousse polymère est perforée.

3. Electrode selon l'une quelconque des revendications 1 ou 2, **caractérisée en ce que** le recouvrement non collant amovible (9) côté peau, qui est retiré directement avant l'application, recouvre complètement au moins la section (4) se trouvant en dehors du recouvrement (16) de la languette de prise et est pourvu, sur sa face tournée vers la colle de contact, d'un revêtement antiadhésif.

4. Electrode selon l'une quelconque des revendications 1 à 3, **caractérisée en ce que**, sur le côté opposé à la peau, est appliquée, au moins dans la zone de la languette de prise (5), une bande souple supplémentaire (30) qui dépasse au moins dans la zone (25) du point de pliage.

5. Electrode selon l'une quelconque des revendications 1 à 4, **caractérisée en ce que** la bande polymère imprimée (12) collée sur la bande de mousse polymère (8) présente un contour externe (27) plus grand que le contour externe (29) du matériau support.

6. Electrode selon l'une quelconque des revendications 1 à 5, **caractérisée en ce que** la bande polymère imprimée (12) collée sur la bande de mousse polymère (8) présente un contour externe (27') plus petit que le contour externe (29) du matériau support, la bande polymère (12) dépassant de tous côtés au moins du corps de gel conducteur (11) et s'étendant jusqu'à l'élément de prise (6, 7).

7. Electrode selon l'une quelconque des revendications 1 à 6, **caractérisée en ce que** la connexion (26) dans la languette de prise est reliée avec une liaison à bouton pression (6) ou avec une liaison à serrage (7) ou encore avec un cordon à un élément de liaison.

8. Procédé de fabrication d'électrodes biomédicales, en particulier selon l'une quelconque des revendications 1 à 7, par les étapes de procédé suivantes :
a) on imprime une bande polymère (12) optiquement transparente, souple et résistant à la déchirure d'un motif de vernis conducteur (13) de différente densité d'impression par impression en continu (14), la densité d'impression étant modifiée par juxtaposition de motifs individuels (21) et le motif de vernis conducteur (13) ainsi aménagé pour chaque électrode dans la zone (13a) du corps de gel conducteur (11) ayant une densité d'impression réglée de sorte qu'au moins deux tiers de cette zone (13a) soient optiquement transparents, que la zone (13b) de la bande polymère (12), qui forme la transition avec la languette de prise (5), soit pourvue d'un motif de vernis conducteur (13) de densité d'impression plus grande, qui est supérieure d'au moins un tiers à la densité d'impression dans la zone (13a) du corps de gel conducteur (11), et que le motif de vernis conducteur (13) dans la zone (13c), qui se trouve directement autour de l'élément de prise (6, 7) agencé dans la languette de prise (5), ait une densité d'impression qui soit au moins aussi élevée que la densité d'impression dans la zone (13a) du corps de gel conducteur (11),
b) on achemine à un appareil automatique de fabrication travaillant par intermittence ou en continu une bande de mousse polymère (8) essentiellement à pores fermés revêtue sur les deux faces d'une colle de contact, au moins leur face tournée vers la peau étant apprêtée de façon compatible avec la peau, une face étant pourvue d'un film de protection amovible et l'autre face collante libre étant recouverte d'un film de protection amovible, les films de protection étant incisés dans la direction de transformation pour des raisons de technique de fabrication,
c) on ménage dans la bande de mousse polymère (8) recouverte de façon non collante sur les deux faces au moins une ouverture (10) pour le corps de gel conducteur (11),
d) on enlève le film de protection opposé à la peau et tourné vers le bas dans l'appareil automatique de fabrication en dehors des éléments de fixation de l'appareil automatique,
e) on applique sur la surface à présent collante de la bande de mousse polymère (8) la bande polymère (12) fabriquée selon l'étape a) du procédé avec la face vernie tournée vers le haut,
f) ensuite, on monte les éléments de prise (6, 7) pour la connexion externe,
g) on introduit pour fermeture dans la cavité formée par l'ouverture (10) de la bande de mousse polymère (8) et par la bande polymère (12) collée par le bas un corps de gel conducteur optiquement transparent (11) de façon plane par rapport à la face supérieure de la bande de mousse polymère (8),
h) ensuite, on arrache le film de protection tourné vers le haut et se trouvant en dehors des éléments de fixation de l'appareil automatique de la bande de mousse polymère (8) et on pourvoit la partie tournée vers la peau, qui forme la languette de prise (5), d'un recouvrement (16) isolant de l'électricité, souple et résistant au soudage,
i) ensuite, on recouvre au moins les surfaces encore collantes, notamment celle du corps de gel conducteur (11) sur la face tournée vers la peau, d'un film de protection amovible (9), et
j) on découpe finalement par matriçage l'électrode finie (1).

9. Procédé de fabrication d'électrodes biomédicales, en particulier selon l'une quelconque des revendications 1 à 7, par les étapes de procédé suivantes :
a) on imprime une bande polymère (12) optiquement transparente, souple et résistant à la déchirure d'un motif de vernis conducteur (13) de différente densité d'impression par impression individuelle (15), le motif de vernis conducteur (13) pour chaque électrode dans la zone (13a) du corps de gel conducteur (11) ayant une densité d'impression réglée de sorte qu'au moins deux tiers de cette zone (13a) soient optiquement transparents, que la zone (13b) de la bande polymère (12), qui forme la transition avec la languette de prise (5), soit pourvue d'un motif de vernis conducteur (13) de densité d'impression plus grande, qui est d'au moins un tiers plus élevée que la densité d'impression dans la zone (13a) du corps de gel conducteur (11), et que le motif de vernis conducteur (13) dans la zone (13c), qui se trouve directement autour de l'élément de prise (6, 7) agencé dans la languette de prise (5), ait une densité d'impression qui soit au moins aussi élevée que la densité d'impression dans la zone (13a) du corps de gel conducteur (11), les surfaces de contact direct de la bande polymère (12) avec la bande de mousse polymère (8) comme matériau support étant pourvues d'une colle de contact et la face revêtue de colle de contact de la bande polymère (12) étant recouverte d'une bande de protection (28), pour former des étiquettes individuelles conductrices finies (24) permettant un stockage intermédiaire à partir de la bande polymère (12),
b) on introduit dans un appareil automatique de fabrication une bande de mousse polymère (8) essentiellement à pores fermés formant le matériau support, qui n'est revêtue d'une colle de contact que sur la face tournée vers la peau, cette face étant recouverte d'un film de protection et étant tournée vers le haut, et le film de protection est incisé le long des éléments de retenue de l'appareil automatique,
c) on ménage dans la bande de mousse polymère (8) au moins une ouverture (10) pour le corps de gel conducteur (11),
d) on prépare une étiquette individuelle finie (24), telle que préfinie selon l'étape a) du procédé, on la place en position correcte en dessous de la face inférieure de la bande de mousse polymère (8) et on la colle de manière durable au moyen d'éléments presseurs à la bande de mousse polymère (8), le motif de vernis conducteur (13) étant tourné vers le haut dans la direction de l'ouverture (10) ménagée pour le corps de gel conducteur (11),
e) on introduit pour fermeture dans la cavité formée par l'ouverture (10) de la bande de mousse polymère (8) et par l'étiquette individuelle collée dessus (24) un corps de gel conducteur optiquement transparent (11) de façon plane par rapport à la face supérieure de la bande de mousse polymère (8),
f) ensuite, on monte les éléments de prise (6, 7) pour la connexion externe,
g) puis, on retire le film de protection appartenant au matériau support (8), on applique au rouleau un matériau de recouvrement (16) en forme de bande, non collant côté peau, souple, isolant de l'électricité et résistant au soudage sur la face inférieure de la languette de prise (5), et on applique un film de protection (9) recouvrant la face inférieure de l'électrode, qui recouvre le corps de gel conducteur (11) et la couche de colle de contact du matériau support (8), et
h) on découpe finalement par matriçage l'électrode finie (2, 3).

10. Procédé de fabrication d'électrodes biomédicales, en particulier selon l'une quelconque des revendications 1 à 7, par les étapes de procédé suivantes :
a) on introduit dans un appareil automatique de fabrication fonctionnant par intermittence ou en continu, avec un certain décalage dans le temps, tout d'abord une bande polymère (12) optiquement transparente, souple et résistant à la déchirure, puis une bande de mousse polymère (8) essentiellement à pores fermés formant le matériau support, la bande de mousse polymère (8) étant revêtue de colle sur les deux faces et au moins la face tournée vers la peau étant apprêtée de façon compatible avec celle-ci, une face étant pourvue d'un film de protection amovible et l'autre face collante libre étant recouverte d'un film de protection amovible, et les films de protection étant incisés dans la direction de la transformation pour des raisons de technique de fabrication,
b) on imprime dans l'appareil automatique de fabrication la bande polymère (12) d'un motif de vernis conducteur (13) de différente densité d'impression par impression individuelle (15), le motif de vernis conducteur (13) pour chaque électrode ayant dans la zone (13a) du corps de gel conducteur (11) une densité d'impression réglée de sorte qu'au moins deux tiers de cette zone (13a) soient optiquement transparents, que la zone (13b) de la bande polymère (12), qui forme la transition avec la languette de prise (5), soit pourvue d'un motif de vernis conducteur (13) de densité d'impression plus grande, qui est au moins d'un tiers supérieure à la densité d'impression dans la zone (13a) du corps de gel conducteur (11), et que le motif de vernis conducteur (13) dans la zone (13c), qui se trouve directement autour de l'élément de prise (6, 7) agencé dans la languette de prise (5), ait une densité d'impression qui soit au moins aussi élevée que la densité d'impression dans la zone (13a) du corps de gel conducteur (11), et les motifs de vernis conducteur respectifs (13) étant agencés à une distance définie l'un de l'autre en fonction de l'affectation de l'électrode,
c) on ménage dans la bande de mousse polymère (8) recouverte de façon non collante sur les deux faces au moins une ouverture (10) pour le corps de gel conducteur (11),
d) on enlève le film de protection opposé à la peau et tourné vers le bas dans l'appareil automatique de fabrication en dehors des éléments de fixation de l'appareil automatique,
e) on applique sur la surface à présent collante de la bande de mousse polymère (8) la bande polymère (12) avec la face vernie tournée vers le haut en fonction de l'affectation de l'électrode,
f) ensuite, on monte les éléments de prise (6, 7) pour la connexion externe,
g) on introduit pour fermeture dans la cavité formée par l'ouverture (10) de la bande de mousse polymère (8) et par la bande polymère (12) collée par le bas un corps de gel conducteur optiquement transparent (11) de façon plane par rapport à la face supérieure de la bande de mousse polymère (8),
h) ensuite, on retire le film de protection tourné vers le haut et se trouvant en dehors des éléments de fixation de l'appareil automatique, la partie tournée vers la peau de la languette de prise (5) étant pourvue d'un recouvrement (16) non collant, isolant de l'électricité, souple et résistant à la soudure,
i) ensuite, on recouvre au moins les surfaces encore collantes, notamment celle du corps de gel conducteur (11), sur la face tournée vers la peau, d'un film de protection amovible (9), et
j) on découpe finalement par matriçage l'électrode finie (2, 3).

11. Electrode selon au moins l'une quelconque des revendications précédentes, **caractérisée en ce qu'**elle est constituée d'un matériau laissant passer les rayons X.
